# EUROPEAN PATENT SPECIFICATION

(11) **EP 2 915 524 B1**
(45) Date of publication and mention of the grant of the patent: **06.09.2017**
(21) Application number: 13852059.8
(22) Date of filing: 07.10.2013
(51) Int. Cl.: A61K 8/894, A61K 8/89, A61Q 17/04, A61K 8/41, A61K 8/58, A61K 8/26, A61K 8/31, A61K 8/891, A61K 8/06

(54) **SUNSCREEN COSMETIC**
SONNENSCHUTZ-KOSMETIKUM
COSMÉTIQUE D'ÉCRAN SOLAIRE

(30) Priority: 30.10.2012 JP 2012239361
(43) Date of publication of application: 09.09.2015
(73) Proprietor: Shiseido Company, Ltd., Chuo-ku Tokyo 104-0061 (JP)
(72) Inventor: SASAKI Kazutaka, Yokohama-shi Kanagawa 224-8558 (JP)
(74) Representative: Ter Meer Steinmeister & Partner
(86) International application number: PCT/JP2013/077204
(87) International publication number: WO 2014/069173

(56) References cited:
- WO-A1-2012/026235
- JP-A- 2009 084 171
- JP-A- 2009 269 881
- JP-A- 2010 059 136
- SHIN ETSU: "Shin Etsu Unique Materials. Silicone Products for Personal Care", INTERNET CITATION, 1 February 2008 (2008-02-01), page 20pp, XP007906619, Retrieved from the Internet: URL:http://www.shinetsusilicones.com/SESA% 20Personal%20Care%20Unique%20Material.pdf [retrieved on 2008-12-12]
- "ABIL EM 90. Emulsifier for the formulation of cosmetic W/O creams and lotions", INTERNET CITATION, 17 March 2006 (2006-03-17), XP007906324, Retrieved from the Internet: URL:http://web.archive.org/web/20060317085 157/http://www.degussa-personal-care.com/p ublic/products/pdf/DS_Abil_EM_90_e.pdf [retrieved on 2008-11-11]

## Description

### TECHNICAL FIELD

The present invention relates to a sunscreen cosmetic. More specifically, it relates to a sunscreen cosmetic that has very high water resistance, superior emulsification stability, and superior texture during use.

### BACKGROUND ART

In the field of emulsified sunscreen cosmetics, a sunscreen cosmetic that has very high water resistance, superior emulsification stability, and superior texture during use is required.

For example, Patent Document 1 discloses an emulsified sunscreen cosmetic that manifests superior water repellency, less re-emulsification when exposed to water resulting in long-lasting coverage, and superior texture during use, emulsification stability, and safety. The sunscreen cosmetic described in Patent Document 1, instead of using a conventional surfactant, uses an alkyl acrylate/alkyl methacrylate copolymer to emulsify the oil based substance and also uses a water resistant film forming agent to provide an emulsified sunscreen cosmetic that manifests superior water resistance.

Commonly, water-in-oil emulsified preparations, compared with oil-in-water preparations, are known to have a higher water repellency and water resistance, but the sunscreen cosmetic described in Patent Document 1 indicates an improvement in water resistance focused on oil-in-water emulsified preparations. However, regarding water-in-oil emulsified preparations, it is pointed out "they are sticky and it is difficult to achieve a refreshing texture, and there are problems in terms of stability" and water resistance of water-in-oil emulsified preparations has not been improved.

Also, Patent Document 2 discloses a sunscreen cosmetic that sustains superior texture during use and manifests superior water resistance, prepared by blending in (Diphenylmethicone/ vinyldiphenyldimethicone/ silsesquioxane) crosspolymer, a specific high polymer silicone, and an ultraviolet absorbent having the maximum absorbance wave length in the UV-A region to reduce the sticky sensation and squeaky sensation while maintaining a high ultraviolet protection effect.

However, the only combination that can achieve both the texture during use and water resistance is (Diphenylmethicone/ vinyldiphenyldimethicone/ silsesquioxane) crosspolymer and highly polymerized dimethicone; and there is a problem in that blending highly polymerized dimethicone in a sunscreen cosmetic results in a heavy texture during use, limiting the range of texture during use.

### {Prior art documents}

### {Patent Documents}

Patent Document 1: JP H07-89834 A
Patent Document 2: JP 2010-90074 A

### DISCLOSURE 0F INVENTION

### TECHNICAL PROBLEM

The object of the invention of the present application is to provide a sunscreen cosmetic that manifests a high water resistance and also superior texture during use and superior emulsification stability by blending in a specific polysiloxane copolymer as an essential ingredient.

### TECHNICAL SOLUTION

That is, the present invention provides a sunscreen cosmetic comprising the following components A through E, as defined in present claim 1:
A. Ultraviolet protection agent
B. Volatile oil component
C. Cetyl dimethicone copolyol
D. (Acrylate/stearyl acrylate/dimethicone methacrylate) copolymer
E. Organically modified clay mineral

The aforementioned sunscreen cosmetic is a water-in-oil emulsified cosmetic.

Furthermore, the content of said C. Cetyl dimethicone copolyol is 0. 1-5 wt%, the content of said D. (Acrylate/ stearyl acrylate/ dimethicone methacrylate) copolymer is 0.05-2 wt%.

### ADVANTAGEOUS EFFECTS 0F THE INVENTION

The sunscreen cosmetic of the present invention has excellent water resistance.

Also, the sunscreen cosmetic of the present invention has excellent emulsification stability.

Furthermore, the sunscreen cosmetic of the present invention has an excellent texture during use (not sticky and not oily).

Also, the sunscreen cosmetic of the present invention is a sunscreen cosmetic that is less likely to cause a white haze even when exposed to sweat and/or water.

### BEST MODE FOR CARYING OUT THE INVENTION

Details of the present invention are described below.

### "A. Ultraviolet protection agent"

In the present invention, the ultraviolet protection agent is an ultraviolet absorbent or an ultraviolet absorbent and ultraviolet scattering agent, said ultraviolet scattering agent being fine particle titanium dioxide or fine particle zinc oxide having an average particle size of 10 nm - 100 nm.

In the present invention, it is preferable to blend in both an ultraviolet absorbent and an ultraviolet scattering agent.

Selection of the ultraviolet absorbent used in the present invention is not limited; examples include the following compounds:

### (1) Benzoic acid ultraviolet light absorbents

For example, paraminobenzoic acid (hereafter abbreviated as PABA), PABA monoglycerin ester, N,N-dipropoxy PABA ethyl ester, N,N-diethoxy PABA ethyl ester, N, N-dimethyl PABA ethyl ester, N,N-dimethyl PABA butyl ester, and N,N-dimethyl PABA ethyl ester.

### (2) Anthranilic acid ultraviolet light absorbents

For example, homo mentyl-N-acetyl anthranilate.

### (3) Salicylic acid ultraviolet light absorbents

For example, amyl salicylate, mentyl salicylate, homo mentyl salicylate, octyl salicylate, phenyl salicylate, benzyl salicylate, and p-isopropanol phenyl salicylate).

### (4) Cinnamic acid ultraviolet light absorbents

For example, ethylhexyl methoxycinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-*α*-cyano-*β*- phenyl cinnamate, 2-ethylhexyl-*α*-cyano-*β*-phenyl cinnamate, and glyceryl mono-2-ethyl hexanoyl-diparamethoxy cinnamate.

### (5) Triazine ultraviolet light absorbents

### Examples include bisresorsinyl triazine.

More specifically, bis{[4-(2-ethylhexyloxy)-2-hydroxy]phenyl}-6-(4-methoxyphenyl)-1,3,5-triazine, and 2,4,6-Tris {4-(2-ethylhexyloxycarbonyl)-anilino}-1,3,5-triazine.

### (6) Other ultraviolet light absorbents

For example, 3-(4'-methylbenzylidene)-d,l-camphor, 3-benzylidene-d,l-camphor, 2-phenyl-5-methyl benzoxazol, 2,2'-hydroxy-5-methylphenyl benzotriazol, 2-(2'-hydroxy-5'-t-octylphenyl) benzotriazol; 2-(2'-hydroxy-5'-methylphenyl benzotriazol), dianisoylmethane, 4-methoxy-4'-t-butyl dibenzoyl-methane, and 5-(3,3-dimethyl-2-norbornylidene)-3-pentane-2-one. Also, pyridazine derivatives such as dimorpholinopyridazinone.

As regards the ultraviolet scattering agent fine particle titanium dioxide and fine particle zinc oxide have an average particle size of 10-100 nm, more preferably 10-50 nm. The average particle size is measured with a usual method such as the number average diameter derived from image analysis of transmission electron microscope images.

Also, in the present invention, it is preferable to use hydrophobized zinc oxide and/or hydrophobized titanium dioxide.

The method of hydrophobizing is not limited in particular; the treatment can be done with a prior art method. Examples include a treatment in which silicones such as methylhydrogen polysiloxane, methylhydrogen polysiloxane/dimethyl polysiloxane copolymer, and dimethyl polysiloxane are used, a treatment in which silane compounds such as octyltriethoxysilane and hexyltrimethoxysilane are used, a treatment in which a fatty acid such as palmitic acid or stearic acid is used, a metal soap treatment in which an alkali metal salt or alkali earth metal salt of said fatty acid is used, and a fluorine treatment in which diethanolamine perfluoroalkylphosphate, perfluoroalkyltrimethoxysilane, etc. are used.

The blend ratio of the ultraviolet protection agent (ultraviolet absorbent and/or ultraviolet scattering agent) is 5-45 wt%, preferably 5-40 wt%, more preferably 5-35 wt%, relative to the total amount of the sunscreen cosmetic.

### "B. Volatile oil component"

For the volatile oil component used in the present invention, low polymerization degree dimethylpolysiloxane and cyclic polysiloxane (for example, octamethylcyclotetrasiloxane, decamethylcyclopentasiloxane, and dodecamethylcyclohexasiloxane), isohexadecane, and fluorine-based oil components, for example, can be used.

The volatile oil component is an ingredient that constitutes the oil phase when the sunscreen cosmetic of the present invention is prepared as an emulsified composition.

The blend ratio of the volatile oil component is 1-40 wt%, preferably 5-35 wt%, more preferably 10-30 wt%, relative to the total amount of the sunscreen cosmetic.

### "C. Cetyl dimethicone copolyol"

In the present invention, commercial products can be used for the cetyl dimethicone copolyol. For example, a commercial product named "ABIL EM90 (from Degussa)" can be used preferably.

In the present invention, it is an ingredient that functions as a surfactant when preparing the water-in-oil emulsified sunscreen cosmetic.

For example, in Table 2 shown later, it functions as a surfactant for the water-in-oil emulsified sunscreen cosmetic in Example 1 and achieves superior emulsification stability. In contrast, Comparative example 1 and Comparative example 2 use PEG-9 polydimethylsiloxyethyl dimethicone and PEG-10 dimethicone, respectively, for the surfactant and therefore emulsification stability is not achieved.

The blend ratio of the cetyl dimethicone copolyol is 0. 1-5 wt%, preferably 0. 1-4 wt%, more preferably 0.1-3 wt%, relative to the total amount of the sunscreen cosmetic.

### "D. (Acrylate/stearyl acrylate/dimethicone methacrylate) copolymer"

(Acrylate/stearyl acrylate/dimethicone methacrylate) copolymer used in the present invention is also called stearyl-modified acrylate silicone; and commercially available products can be used for it. For example, a commercial product named "KP-561P (from Shin-Etsu Chemical Co., Ltd.)" can be used preferably.

The blend ratio of the (acrylate/stearyl acrylate/dimethicone methacrylate) copolymer is 0.05-2 wt%, more preferably 0.05-1 wt%, relative to the total amount of the sunscreen cosmetic.

### "E. Organically modified clay mineral"

The organically modified clay mineral used in the present invention is used as an emulsion aid when preparing the sunscreen cosmetic of the present invention as an emulsified composition. The organically modified clay mineral is a type of colloidal aluminum silicate hydrate that has a three-layer structure that is prepared by modifying a clay mineral with a quaternary ammonium salt cationic surfactant. For example, organically modified bentonite and organically modified hectorite can be used.

Specific examples include dimethyldistearyl ammonium hectorite, dimethyl alkyl ammonium hectorite, benzyl dimethyl stearyl ammonium hectorite, and aluminum magnesium silicate treated with distearyl dimethyl ammonium chloride. Preferable commercially available examples to be blended in include Benton 27 (hectorite treated with benzyl dimethyl stearyl ammonium chloride, available from Nationalred Co.) and Benton 38 (hectorite treated with distearyl dimethyl ammonium chloride, available from Nationalred Co.).

The blend ratio of the organically modified clay mineral is 0.1-5 wt%, preferably 0.1-4 wt%, more preferably 0.1-3 wt%, relative to the total amount of the sunscreen cosmetic.

The formulation form of a sunscreen cosmetic containing the aforementioned essential ingredients is not limited; it is preferable to prepare it as a water-in-oil emulsified sunscreen cosmetic such as a cream and emulsion.

The surfactant used to prepare the water-in-oil emulsified sunscreen cosmetic is the essential ingredient "C. Cetyl dimethicone copolyol"; it is difficult to achieve emulsification stability with other surfactants.

Also, as an essential ingredient of the water-in-oil emulsified sunscreen cosmetic, "F. Water" is further blended in as the water phase. It is preferable to blend in ethanol, glycerin and such in the water phase.

### "(F) Water"

Water used in the present invention is an ingredient that constitutes the water phase of the water-in-oil emulsified sunscreen cosmetic. Its blend ratio is 5-30 wt% relative to the total amount of the water-in-oil emulsified sunscreen cosmetic.

In the water-in-oil emulsified sunscreen cosmetic of the present invention, the mass ratio of the water phase (including water soluble ingredients dissolved in water) and the oil phase (including ingredients dissolved or dispersed in the oil component) is preferably in the range of (water phase) : (oil phase) = 3:7 - 1:9.

In addition to the aforementioned essential ingredients, other ingredients usually used in cosmetics can be blended in as appropriate into the sunscreen cosmetic of the present invention as long as the effect of the present invention is not adversely affected; examples of such ingredients include oil components, humectants, thickeners, powders, alcohols, natural polymers, synthetic polymers, sugars, antioxidants, buffers, various extracts, stabilizers, preservatives, pigments, and perfumes.

### EXAMPLES

The present invention is described in detail through Examples below, but the invention shall not be limited to them. The blend ratios are in relation to the total amount and in mass-percentage units unless specified otherwise.

The oil phase ingredients of the water-in-oil emulsified sunscreen cosmetic in Table 1 and emulsions that were the water-in-oil emulsified sunscreen cosmetics of Table 2 were prepared with a conventional method and water resistance and emulsification stability were evaluated.

The oil phase ingredients of the water-in-oil emulsified sunscreen cosmetic shown in Table 1 are to be mixed with the water phase ingredients with a conventional method to form a water-in-oil emulsified sunscreen cosmetic. The oil phase ingredients in Table 1 were evaluated for their water resistance.

### "Evaluation of water resistance 1"

The water resistance of the oil phase film itself, i.e. the outer phase, was evaluated by investigating the oil ingredients of Table 1 (said oil phase ingredients were to become the outer phase of the water-in-oil emulsified sunscreen cosmetic) that were used when preparing the water-in-oil emulsified sunscreen cosmetic.

### <Absorbance before water exposure>

The oil phase ingredients of Test example 1 (oil phase ingredients that mix with the water phase to form the water-in-oil emulsified sunscreen cosmetic of the present invention) and Comparative test examples 1-5 (oil phase ingredients that do not form the water-in-oil emulsified sunscreen cosmetic of the present invention after mixing with the water phase because of the absence of the (acrylate/stearyl acrylate/dimethicone methacrylate) copolymer), 2 mg/cm² each, were applied on PMMA plates and a spectrophotometer (Hitachi U-4100) was used to measure the absorbance in the range from 280 nm to 400 nm.

### <Absorbance after water exposure>

The aforementioned PMMA plates were immersed in water for water exposure at 29°C for 30 minutes; after drying, the absorbance from 280 nm to 400 nm was measured with the spectrophotometer (Hitachi U-4100).

### <Evaluation of water resistance 1>

The integral value ratios of the aforementioned absorbance at 280-400 nm before and after the water exposure were calculated and the water resistance was evaluated as follows:
○ : The integral value ratio is over 90%.
△ : The integral value ratio is over 80% and not more than 90%.
× : The integral value ratio is not more than 80%.

### "Evaluation of water resistance 2"

The water-in-oil emulsified sunscreen cosmetics (emulsions) of Table 2 were applied on arms at 2 mg/cm² and exposed to water for 30 minutes; after drying, the water repellency was evaluated visually.

### Evaluation of the emulsification stability

○ : Water repellency is maintained.
× : Water repellency is not maintained.

### "Emulsification stability"

The water-in-oil emulsified sunscreen cosmetics (emulsions) of Examples and Comparative examples in Table 2 were each put into a glass container and rotated at 45 rpm for 4 hours; the emulsified particles were visually observed for coalescence and evaluated based on the following criteria.
○ : No coalescence of the emulsified particles was observed.
× : Coalescence of the emulsified particles was observed.

### <The oil phase ingredients that constitute the water-in-oil emulsified sunscreen cosmetic>

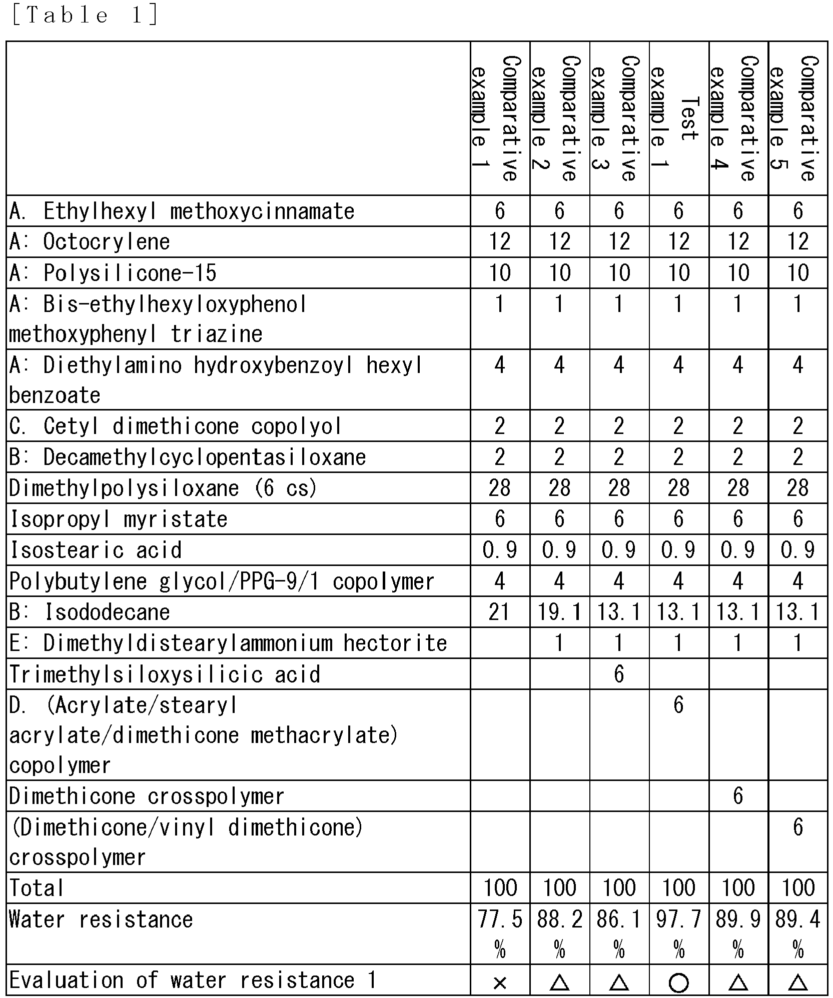

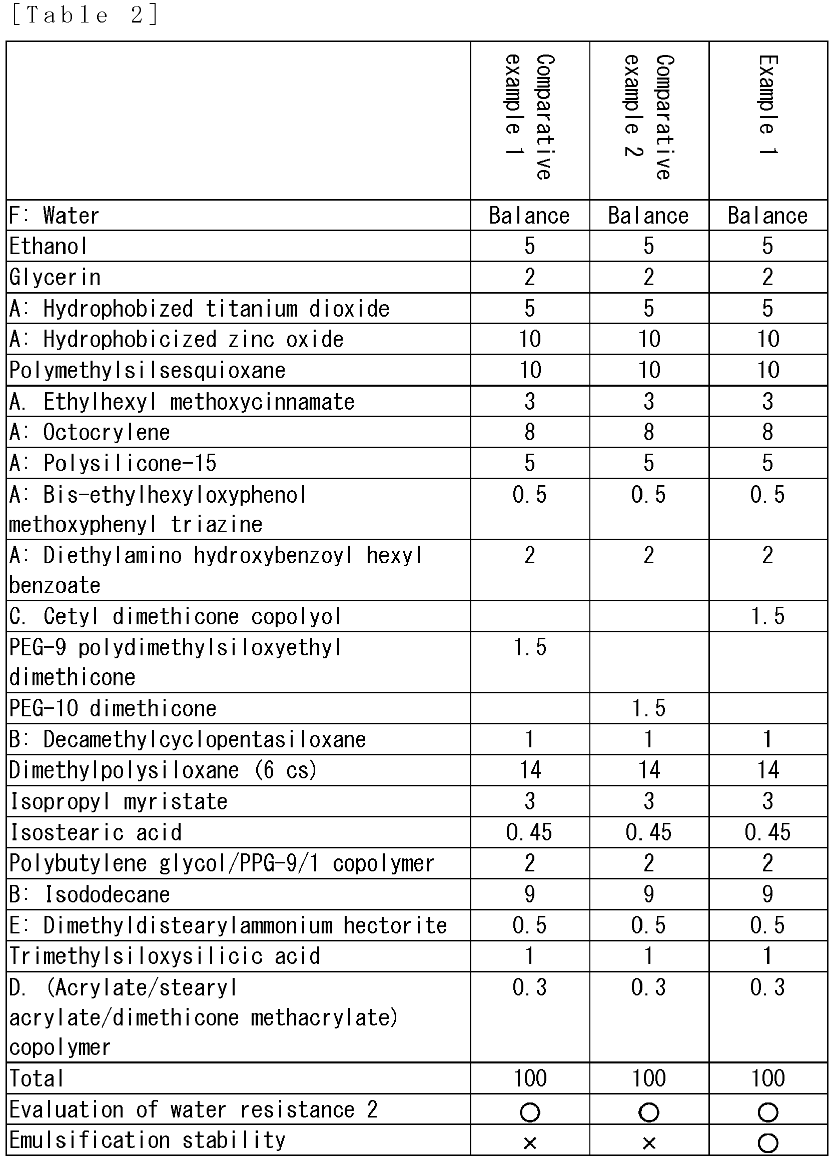

The results shown in Table 1 and Table 2 indicate that the sunscreen cosmetic of the present invention manifests excellent water resistance and emulsification stability.

Furthermore, the sunscreen cosmetic of Example 1 is a sunscreen cosmetic that is superior in terms of texture during use (not sticky, not oily) and does not cause a white haze even when exposed to sweat and/or water.

Other Examples of the present invention are described below. Every one of them is a sunscreen cosmetic that manifests excellent water resistance and emulsification stability, is superior in terms of texture during use (not sticky, not oily) and does not cause a white haze even when exposed to sweat and/or water.

### Example 2: Sunscreen cream (W/0 cream)

| Ingredient | wt% |
|---|---|
| (1) Fine particle hydrophobized zinc oxide | 20 |
| (2) Fine particle titanium dioxide | 2 |
| (3) Ethylhexyl methoxycinnamate | 7 |
| (4) Decamethylcyclopentasiloxane | 10 |
| (5) Isopropyl myristate | 5 |
| (6) Mineral oil | 1 |
| (7) Carboxy-modified silicone | 2 |
| (8) (Acrylate/stearyl acrylate/dimethicone methacrylate) copolymer | 0.8 |
| (9) Cetyl dimethicone copolyol | 1 |
| (10) Organically modified bentonite | 1 |
| (11) Ion-exchanged water | Balance |
| (12) Glycerin | 2.5 |
| (13) 1,3-butylene glycol | 5 |
| (14) Alcohol | 5 |
| (15) Phenoxyethanol | 0.5 |

Preparation method: (1)-(10) were mixed and dispersed. The water phase, which was (11)-(15) mixed and dissolved, was then added and mixed to obtain the target sunscreen cream.

### Example 3: Sunscreen lotion (two layer type W/0 emulsion)

| Ingredient | wt% |
|---|---|
| (1) Decamethylcyclopentasiloxane | 20 |
| (2) Isohexadecane | 10 |
| (3) Dimethylpolysiloxane (6 cs) | 5 |
| (4) Cetyl dimethicone copolyol | 0.8 |
| (5) Organically modified bentonite | 0.5 |
| (6) Octocrylene | 3 |
| (7) Ethylhexyl methoxycinnamate | 5 |
| (8) Fine particle hydrophobized zinc oxide | 12 |
| (9) Fine particle titanium dioxide | 5 |
| (10) Poly methyl methacrylate spherical powder | 3 |
| (11) (Acrytate/stearyt acrylate/dimethicone methacrylate) copolymer | 1 |
| (12) Ion-exchanged water | Balance |
| (13) 2-amino-2-methyl-1,3-propandiol | 1 |
| (14) Phenylbenzimidazolesulfonic acid | 2 |
| (15) Glycerin | 2 |
| (16) 1,3-butylene glycol | 3 |
| (17) Paraben | 0.2 |

Preparation method: (1)-(11) were mixed and dispersed. The water phase, which was (12)-(17) mixed and dissolved, was then added and mixed to obtain the target sunscreen lotion.

### Example 4: Sunscreen lotion (two layer type W/0 emulsion)

| Ingredient | wt% |
|---|---|
| (1) Isohexadecane | 17 |
| (2) Dimethylpolysiloxane (6 cs) | 5 |
| (3) Glyceryl tri-(caprylate-caprate) | 5 |
| (4) Isostearic acid | 1 |
| (5) Cetyl dimethicone copolyol | 2 |
| (6) Organically modified bentonite | 0.4 |
| (7) Organopolysiloxane elastomer spherical powder | 10 |
| (8) Fine particle hydrophobized zinc oxide | 15 |
| (9) (Acrylate/stearyl acrylate/dimethicone methacrylate) copolymer | 0.2 |
| (10) Ion-exchanged water | Balance |
| (11) Tranexamic acid | 2 |
| (12) EDTA | 0.2 |
| (13) Xylitol | 3 |
| (14) 1,3-butylene glycol | 5 |
| (15) Phenoxyethanol | 0.5 |

Preparation method: (1)-(9) were mixed and dispersed. The water phase, which was (10)-(15) mixed and dissolved, was then added and mixed to obtain the target sunscreen lotion.

### INDUSTRIAL APPLICABILITY

The sunscreen cosmetic of the present invention is a sunscreen cosmetic that has very high water resistance, superior emulsification stability, and superior texture during use.

## Claims

1. A sunscreen cosmetic which is a water-in-oil emulsified cosmetic having excellent water resistance and excellent emulsification stability, comprising the following components (A) through (E):
(A) ultraviolet protection agent which is an ultraviolet absorbent or an ultraviolet absorbent and ultraviolet scattering agent, said ultraviolet scattering agent is fine particle titanium dioxide or fine particle zinc oxide having an average particle size of 10 nm - 100 nm, the content of (A) is 5-45 wt%,
(B) volatile oil component, the content of (B) is 1-40 wt%,
(C) cetyl dimethicone copolyol, the content of (C) is 0.1-5 wt%,
(D) (acrylate/stearyl acrylate/dimethicone methacrylate) copolymer, the content of (D) is 0.05-2 wt%,
(E) organically modified clay mineral.

## Patentansprüche

1. Sonnenschutzkosmetikum, das ein Wasser-in-Öl-emulgiertes Kosmetikum mit einer ausgezeichneten Wasserbeständigkeit und ausgezeichneter Emulgierungsstabilität ist, umfassend die folgenden Komponenten (A) bis (E):
(A) Ultraviolett-Schutzmittel, das ein Ultraviolett-Absorptionsmittel oder ein Ultraviolett-Absorptionsmittel und Ultraviolett-Streuungsmittel ist, wobei das Ultraviolett-Streuungsmittel feinteiliges Titandioxid oder feinteiliges Zinkoxid mit einer durchschnittlichen Teilchengröße von 10 nm - 100 nm ist, wobei der Gehalt an (A) 5-45 Gew.-% beträgt,
(B) flüchtige Ölkomponente, wobei der Gehalt an (B) 1-40 Gew.-% beträgt,
(C) Cetyldimethiconcopolyol, wobei der Gehalt an (C) 0,1-5 Gew.-% beträgt,
(D) (Acrylat/Stearylacrylat/Dimethiconmethacrylat)-Copolymer, wobei der Gehalt an (D) 0,05-2 Gew.-% beträgt,
(E) organisch modifiziertes Tonmineral.

## Revendications

1. Un cosmétiques à écran solaire qui est un cosmétique émulsifié eau-dans-huile ayant une excellente résistance à l'eau et un excellente stabilité d'émulsification, comprenant les composants suivants (A) à (E):
(A) agent de protection contre les rayons ultraviolets qui est un absorbant de rayons ultraviolets et un agent de dispersement de rayons ultraviolets, l'agent de dispersement de rayons ultraviolets est du dioxyde de titane en particules fines ou de l'oxyde de zinc en particules fines ayant une taille de particules de 10 nm - 100 nm, la teneur en (A) est de 5-45% en poids,
(B) composant d'huile volatile, la teneur en (B) est de 1-40% en poids,
(C) cétyl-diméthicone-copolyol, la teneur en (C) est de 0,1-5% en poids,
(D) copolymère de (acrylate / acrylate de stéaryle / méthacrylate de diméthicone), la teneur en (D) est de 0,05-2% en poids,
(E) minéral d'argile modifié de manière organique.
